# EUROPÄISCHE PATENTSCHRIFT

(11) **EP 3 138 522 B1**
(45) Veröffentlichungstag und Bekanntmachung des Hinweises auf die Patenterteilung: **04.11.2020**
(21) Anmeldenummer: 15183777.0
(22) Anmeldetag: 03.09.2015
(51) Int. Cl.: A61B 18/14

(54) **INSTRUMENT ZUM FASSEN, TRENNEN UND/ODER KOAGULIEREN VON BIOLOGISCHEM GEWEBE**
INSTRUMENT FOR MOUNTING, SEPARATING AND/OR COAGULATION OF BIOLOGICAL TISSUE
INSTRUMENT DE PREHENSION, DE SEPARATION ET/OU DE COAGULATION DE TISSUS BIOLOGIQUES

(43) Veröffentlichungstag der Anmeldung: 08.03.2017
(73) Patentinhaber: Erbe Elektromedizin GmbH, 72072 Tübingen (DE)
(72) Erfinder: Mayer, Volker, Dr., 72072 Tübingen (DE); Weiler, Rolf, 72127 Kusterdingen (DE)
(74) Vertreter: Rüger Abel Patentanwälte PartGmbB

(56) Entgegenhaltungen:
- EP-A1- 2 959 854
- CN-A- 104 382 648
- US-A1- 2004 049 185
- US-A1- 2009 234 355
- US-A1- 2013 046 295

## Beschreibung

Die Erfindung betrifft ein manuell oder maschinell geführtes Instrument, mit dem biologisches Gewebe gefasst, koaguliert und getrennt oder nur koaguliert werden kann.

Ein solches als Schere ausgebildetes Instrument ist aus der US 2004/0049185 A1 bekannt. Das Instrument weist zwei Branchen auf, die über ein Scharnier miteinander verbunden sind und dadurch aufeinander zu und voneinander weg geschwenkt werden können. Die Branchen können zwischen einander Gewebe fassen. Beide Branchen weisen jeweils einen etwa U-förmigen Querschnitt auf, wobei die Enden der Schenkel des U-förmigen Querschnitts die Koagulationselektroden bilden. Diese koagulieren dazwischen gefasstes Gewebe, wenn die beiden Branchen an die Pole einer entsprechenden Stromquelle angeschlossen sind.

Eine der Branchen weist zusätzlich eine Schneidelektrode auf, die zentrisch zwischen den Koagulationselektroden über diese hinaus vorstehend angeordnet ist. Die gegenüber liegende Branche weist ein Widerlager aus einem elastischen Material auf, in das die Schneidelektrode unter elastischer Verformung des Widerlagers eindringen kann. Damit wird vor der Schneidelektrode liegendes biologisches Material an die Schneidelektrode angedrückt.

Weiter ist aus der US 2005/ 0 171 533 A1 ein ähnliches Instrument mit zwei Branchen, die jeweils Koagulationselektroden tragen, und mit einer Schneidelektrode bekannt, die in einer der Branchen zwischen zwei Koagulationselektroden angeordnet ist. In der gegenüber liegenden Branche ist eine Nut zur Aufnahme der Schneidelektrode vorgesehen. Am Boden der Nut ist ein Isolator aus elastischem Material angeordnet.

Bei den Instrumenten der oben genannten Bauart findet der Schneidvorgang des gefassten Gewebes zwischen der Schneidelektrode und dem Widerlager statt. Dabei kommt es darauf an, dass das Gewebe durch Einwirkung des von der Schneidelektrode ausgehenden elektrischen Stroms sicher und vollständig durchtrennt wird, wobei eine mechanische Schneidwirkung der Schneidelektrode nicht gewünscht ist. Bei der praktischen Anwendung unterliegt die Menge bzw. Dicke sowie die Beschaffenheit des zwischen den Branchen gefassten Gewebes gewissen Variationen. Dennoch soll trotz dieser wechselnden Bedingungen jeweils ein sauberer Schnitt des Gewebes und eine gute Koagulation der erzeugten Gewebesäume erreicht werden. Um eine gute Koagulation zu erreichen, muss das Gewebe mit relativ hoher Kraft zwischen den Koagulationselektroden gefasst sein. An der Schneidelektrode ist hingegen eine deutlich geringere Kraft gewünscht.

Davon ausgehend ist es Aufgabe der Erfindung, ein Konzept für ein maschinell oder manuell geführtes Instrument anzugeben, mit dem sich die genannten Anforderungen erfüllen lassen.

Diese Aufgabe wird mit dem Instrument nach Anspruch 1 gelöst:

Das Instrument weist eine erste Branche mit mindestens einer ersten Koagulations- bzw. Versiegelungselektrode zur Koagulation biologischen Materials auf. Vorzugsweise sind an der ersten Branche zwei erste Versiegelungselektroden vorgesehen, die sich im Abstand zueinander erstrecken und zwischen denen ein Freiraum ausgebildet ist. In dem Freiraum ist ein vorzugsweise elektrisch isolierender Schneidelektrodenträger angeordnet, der die Schneidelektrode trägt. Dazu kann der Schneidelektrodenträger einen die Versiegelungselektroden überragenden mittigen wandartigen Vorsprung aufweisen, der an seinem freien Ende die Schneidelektrode trägt.

Die zweite Branche weist wenigstens eine zweite Versiegelungselektrode, vorzugsweise zwei im Abstand zueinander angeordnete Versiegelungselektroden auf. In dem zwischen den zweiten Versiegelungselektroden vorhandenen Freiraum ist ein Widerlager angeordnet, das vorzugsweise als gesondert vorgefertigtes Element aus elastischem Material ausgebildet ist. Dieses Widerlager weist mindestens eine nach außen offene Ausnehmung und/oder wenigstens eine Hohlkammer auf. Diese eine oder mehrere Ausnehmungen und/oder Hohlkammern können zur gezielten Einstellung der Federkennlinie des Widerlagers genutzt werden. Damit kann erreicht werden, dass die von dem Widerlager im Ziel-Arbeitsbereich ausgeübte, gegen das Schneidelement bzw. die Schneidelektrode gerichtete Kraft während des gesamten Schneidvorgangs des Gewebes einen Grenzwert von zum Beispiel 1N bis 2N nicht unterschreitet und einen Grenzwert von zum Beispiel 3N oder 4N bzw. 7N oder 8N nicht übersteigt. Im Instrument sind Schneidelement und Widerlager mechanisch über das Gelenk des Maulteils gekoppelt. D.h. über die winklige Anordnung zueinander wird das Widerlager von der Schneidelektrode stets in einer Art Walk-Bewegung komprimiert, d.h. nicht und vorzugsweise nie vollständig parallel komprimiert. Zur Betrachtung des Federverhaltens des Widerlagers und insbesondere dessen Geometrie, ist es deshalb sinnvoll, nur ein sehr kurzes, infinitesimal kleines Segment des Widerlagers zu betrachten. Bei dieser Betrachtung eines Widerlager-Segmentes, ist eine nichtlineare Kraft-Weg-Kennlinie beim Zusammendrücken des Widerlagers erreichbar. Insbesondere kann diese Kraft-Weg-Kennlinie einen Abschnitt mit nahezu konstanter Kraft aufweisen. Eine an diesen Abschnitt angelegte Gerade schneidet den Nullpunkt des Kraft-Weg-Diagramms nicht. Im Idealfall verläuft eine solche Gerade fast parallel zur Weg-Achse (Abszisse). Die Kraft-Weg-Kennlinie hat dementsprechend ein Plateau.

Es können eine oder mehrere Ausnehmungen und/oder eine oder mehrere Hohlkammern im Widerlager vorgesehen sein. Die Ausnehmungen sind nach außen offen, d.h. sie sind nicht allseits von dem Widerlager umschlossen, sondern treten als Öffnung an dessen Oberfläche zutage. Vorzugsweise weist auch die Branche, zumindest einen Durchbruch auf, der mit der Ausnehmung des Widerlagers in Verbindung steht. So können gasförmige oder flüssige Fluide ungehindert aus der Ausnehmung austreten, wenn das Widerlager komprimiert wird. Eine Verhärtung des Widerlagers durch in der Ausnehmung eingeschlossene Flüssigkeiten oder gar durch Koagulation solcher Flüssigkeiten wird vermieden. Alternativ können die Ausnehmungen auch den Querschnitt des Widerlagers ganz oder teilweise durchdringend angeordnet sein.

Bei einer alternativen Ausführung ist es möglich, die Branchen gegenüber den Ausnehmungen des Widerlagers mit einem Verschlusselement, z.B. einem Deckel, auszubilden. In diesem Fall können die oben beschriebenen Fluide entweder weiterhin aus der Ausnehmung des Widerlagers austreten oder das Verschlusselement ist so gestaltet, dass die oben beschriebenen Fluide weiterhin aus den Ausnehmungen des Widerlagers austreten können.

Die großflächig offene Ausnehmung kann das Eindringen und wieder Austreten von Fluiden gestatten. Durch die Größe der Öffnung wird verhindert, dass es beim Betrieb in Flüssigkeit (zum Beispiel Blut) durch die Kompression und Dekompression des Widerlagers zu einer Pumpwirkung der Flüssigkeit kommt. Selbst wenn aufgrund der Wärmefreisetzung an dem Instrument Flüssigkeit zum Beispiel Blut koaguliert und verklumpt, bleiben die Öffnungen nach außen frei, so dass das weitere Ein- und Ausfedern nicht behindert wird.

Als Gewebeauflagefläche wird die Oberfläche des Widerlagers bezeichnet, die dem Schneidelement gegenüberliegt und gegen die das Schneidelement eine Kraft aufbringt, gegebenenfalls auch mit dazwischenliegendem Gewebe. Die Ausnehmung kann durch eine der Gewebeauflagefläche abgewandte Nut oder auch eine Reihe von kurzen voneinander durch Querwände getrennten Nuten ausgebildet sein. Eine solche Nut wird seitlich von Nutwänden begrenzt, die den oberen geschlossenen Teil des Widerlagers tragen, der die Gewebeauflagefläche festlegt. Beim Schließen der Branchen drückt die Schneidelektrode das Widerlager zusammen, womit die unter der Gewebeauflagefläche liegende Ausnehmung zumindest teilweise kollabiert.

Alternativ oder zusätzlich können ein oder mehrere Ausnehmungen vorgesehen sein, die die Gewebeauflagefläche durchsetzen und somit zu der die Schneidelektrode tragenden Branche hin offen sind. Solche Ausnehmungen können insbesondere eine Verbindung mit einer oder mehreren rückseitigen, d.h. von der Gewebeauflagefläche abgewandten Ausnehmungen oder Hohlkammern des Widerlagers ergeben. Die von der Gewebeauflagefläche abgewandten Ausnehmungen oder Hohlkammern sind durch von der Gewebeauflagefläche weg divergierende Wände definiert. Mit dieser Konfiguration lässt sich der gewünschte Kennlinienverlauf besonders leicht und zuverlässig erreichen. Die Wände des Widerlagers können dabei (wie auch bei anderen Ausführungsformen) in Richtung von der Gewebeauflagefläche weg eine konstante Wandstärke aufweisen.

Die an dem Widerlager ausgebildete von der Gewebeauflagefläche weg weisende offene Ausnehmung kann eine oder mehrere Querwände aufweisen. Solche Querwände können eine einheitliche Wandstärke aufweisen oder auch einen Schwächungsbereich haben. Der Schwächungsbereich kann auch ganz entfallen und als Ausschnitt ausgebildet sein. Damit nimmt die Querwand nicht mehr den gesamten Querschnitt der Nut ein. Beim Komprimieren des Widerlagers kann ein teilweiser Kollaps der Ausnehmung stattfinden. Mithilfe der Querwände kann ein Absinken der von dem Widerlager aufgebrachten Kraft aufgrund des teilweisen Kollaps der Ausnehmung verhindert werden. Die Querwände verstärken nicht nur die Gesamtfederkraft des Widerlagers, sondern erzeugen auch die notwendige Kraftkomponente, damit sich das Widerlager auch in Querrichtung bei Druckentlastung wieder rückstellen kann. Die Vorsehung des Schwächungsbereichs oder eines Ausschnitts in den Querwänden vermeidet eine Krafterhöhung, die bei maximaler Kompression auf Anschlag und daraus hervorgerufener Quetschung entstehen kann. Des Weiteren erleichtern die Querwände auch das Handling und die Handhabung des in der Praxis sehr filigranen Widerlagers, denn sie verleihen diesem eine Grundstabilität und verhindern einen Kollaps der gesamten Struktur im nicht eingebauten Zustand.

In eingebautem Zustand ist das Widerlager in der Branche fixiert. Dazu kann das Widerlager seitliche Vorsprünge in Form von Füßen aufweisen, denen komplementäre Ausnehmungen in der Branche zugeordnet sind. Die seitlich abstehenden Vorsprünge fassen in die Ausnehmungen der Branche und halten das Widerlager. Zusätzlich kann ein Halteelement zur Fixierung des Widerlagers vorgesehen sein. Dieses drängt die Füße tragenden Wände des Widerlagers voneinander weg nach außen und sorgt für den festen Sitz der Füße in den Ausnehmungen.

Als Material für das Widerlager wird vorzugsweise ein biokompatibles Silikon mit einer Härte von 50 Shore verwendet, womit sich die gewünschte Federkraft und der oben beschriebene plateauartige Kraftverlauf der Kraft-Weg-Kennlinie leicht erreichen lassen. Sind die Gewebeauflagefläche durchsetzende Ausnehmungen vorgesehen, sind diese vorzugsweise zu beiden Seiten eines streifenförmigen Schneidelektroden-Auftreffbereichs der Gewebeauflagefläche angeordnet. Vorzugsweise ergibt sich eine symmetrische Form des Querschnitts des Widerlagers. Dies gilt insbesondere, wenn die Schneidelektrode mittig auf das Widerlager auftrifft. Entlang des Schneidelektroden-Auftreffbereichs der Gewebeauflagefläche können zu beiden Seiten Stege angeordnet sein, die vorzugsweise oben eben mit dem Schneidelektroden-Auftreffbereich abschließen. Die Stege können an der inneren Flanke der Branche anliegen und bewirken eine Zentrierung des Widerlagers sowohl im Ruhzustand als auch im eingefederten, d.h. ganz oder teilweise zusammengedrückten Zustand. Die Stege können mit gleichen oder wechselnden Abständen und jeweils paarweise auf beiden Seiten oder auch unpaarig angeordnet sein. Es ist auch möglich, dass die Oberfläche der Stege, wannenförmig oder keilförmig ausgebildet ist, um die Zentrierfunktion des Schneidelements zu verstärken. Es ist auch möglich, dass die Gewebeauflagefläche des Schneidelektroden-Auftreffbereichs teilweise gebogen, wannenförmig oder keilförmig ausgebildet sein kann, um diese Zentrierfunktion zu unterstützen.

Durch den vorzugsweise flächigen Anschluss der Stege mit dem Schneidelektroden-Auftreffbereich wird außerdem verhindert, dass das Schneidelement von dem Schneidelektroden-Auftreffbereich seitlich abrutscht, wie es bei asymmetrischer Verformung des Widerlagers sonst vorkommen könnte. Eine solche asymmetrische Verformung hätte auch ein seitliches Abdrängen der Schneidelektrode zur Folge. Die stützenden Stege stabilisieren somit auch die unter Umständen seitlich nachgiebige Schneidelektrode und verhindern deren Abkippen. Damit sorgen sie auch für eine symmetrische Ausbildung der Gewebeaufnahmeräume direkt neben der Schneidelektrode, also zwischen der Schneidelektrode und den Versiegelungselektroden.

Durch das gewünschte Verhalten des Widerlagers, nämlich ein Einknicken der Seitenwände und gewissermaßen ein Umschnappen derselben, muss eine symmetrische Belastung des Widerlagers sichergestellt sein. Eine asymmetrische Belastung könnte sonst zu einer asymmetrischen Verformung des Widerlagers führen. Die stützenden Stege erfüllen hier eine wichtige Funktion, indem sie ein seitliches Abkippen, d.h. schräges Einfedern des Schneidelektroden-Auftreffbereichs bei unsymmetrischer Belastung und somit eine asymmetrische Verformung minimieren. Damit wird auch ein Abgleiten der Schneidelektrode zu einer Seite des Widerlagers hin vermieden. Das Instrument wird außerdem somit auch gegen Kräfte unempfindlich, die bei der praktischen Anwendung auftreten können, wenn beispielsweise das gefasste Gewebe einem seitlichen Zug ausgesetzt ist.

Die die Schneidelektrode tragende Branche weist am proximalen Ende einen Gewebeanschlag auf. Dieser Gewebeanschlag kann in das die Schneidelektrode aufweisende Schneidelement integriert sein und verhindern, dass beim Schneiden von Gewebe Teile desselben in Richtung auf das die Branchen verbindende Gelenk hin hinter die Schneidelektrode des Schneidelements rutschen. Somit wird ein Lochstanzeffekt im Gewebe verhindert und das gesamte gefasste Gewebe auch geschnitten. Das Widerlager kann dazu an seinem proximalen Ende einen Gleitbereich für diesen Gewebeanschlag aufweisen, der vorzugsweise eine zur Gelenkachse konzentrische kreissegmentförmige Ausbildung hat. Alternativ kann der Gewebeanschlag Teil des Widerlagers sein und an dem Schneidelement entlang gleiten. Die proximale Seite des Widerlagers ist jedenfalls so geformt, dass es ungeachtet der Relativposition der Branchen zueinander stets mit dem Gewebeanschlag abschließt.

Mit dem erfindungsgemäßen Instrument kann zwischen Widerlager und Schneidelektrode eine Schnittkraft von ca. 2N bis 4N eingestellt werden, welche bei geschlossenen Branchen auf das zu trennende Gewebe wirkt. Dadurch kann die auf die Schneidelektrode einwirkende Kraft minimiert werden. Zusätzlich wird sichergestellt, dass das Gewebe vollständig getrennt wird. Ein hauchdünner Gewebeteil zwischen den getrennten Gewebeteilen, wie er in der Anwendung gemäß dem Stand der Technik vorkommen kann, wird durch die Sicherstellung und präzise Einstellung der Schnittkraft vermieden. Die Schließkraft der Branchen, die in Branchenmitte etwa 22N betragen kann, kann somit zum weitaus größten Teil für den Versiegelungsprozess genutzt werden. Somit ist die Schnittkraft lediglich so groß wie nötig (ca. 2N bis 4N) wohingegen die an den Versiegelungselektroden vorhandene Kraft so groß wie möglich ist. Das Widerlager bringt durch seine Nachgiebigkeit, und damit Rückfedermöglichkeit, nur etwas mehr als die zum Schneiden notwendige Schneidkraft auf das Gewebe zwischen Widerlager und Schneidelektrode auf. Die übrige Schließkraft entfällt dann auf die Versiegelungselektroden. Der vorgeschriebene Schließprozess des Instruments gilt im Wesentlichen unabhängig davon, ob Gewebe zwischen den Branchen gefasst ist oder nicht.

Mit der genannten Maßnahme werden günstige Stromdichteverhältnisse an der Schneidelektrode geschaffen. Durch das korrekte Andrücken des Gewebes an die Schneidelektrode wird eine hohe Stromdichte an der Schneidelektrode erreicht, ohne das Gewebe mechanisch zu trennen. Die Nachgiebigkeit des Widerlagers sorgt also auch dafür, dass beim Fassen und Manipulieren von Gewebe mit diesem Branchenpaar das Gewebe trotz des schmalen, kantigen Schneidelements so wenig wie möglich traumatisiert wird oder mechanischen Schaden nimmt. Außerdem dient das Widerlager als Toleranzausgleich, der immer sicherstellt, dass der Großteil der Schließkraft auf die Versiegelungselektroden wirkt und nur ein kleinerer auf die Schneidelektrode bzw. das Schneidelement.

Durch die spezifische Ausbildung des Gegenlagers ist auch eine gewisse Beweglichkeit in Branchenlängsrichtung gegeben, die zumindest im Rahmen der durch die elastische Verformung der Branchen und des Schneidelements verursachten Bewegungen liegt. Dies erschließt sich aus der nachfolgenden Kurzbeschreibung des Schließens des Instruments bei zunehmender Schließkraft ohne zwischen den Branchen gefasstes Gewebe:

Zunächst berührt das Schneidelement die Gewebeauflagefläche des Widerlagers unmittelbar am proximalen Ende. Bei weiterer Kraftaufbringung federt das Widerlager unter dem Schneidelement ein, wobei sich auch das Schneidelement und die Branchen geringfügig elastisch verformen können. Durch das Schließen weicht das Widerlager auch etwas in vorzugsweise distaler Branchenlängsrichtung aus.

Das Schneidelement taucht idealerweise senkrecht und mittig in das Widerlager ein. Die Gewebeauflagefläche des Widerlagers baucht ein - es entsteht eine Vertiefung. Die schrägen Wände des Widerlagers wölben sich in Richtung zu den Versiegelungselektroden hin aus. Die Oberseiten der stützenden Stege des Widerlagers legen sich an die Seitenflächen des Schneidelements an und zentrieren es dadurch mittig im Instrument. Dicke und Abstand der stützenden Stege sind dabei so gewählt, dass die dazwischen vorhandenen Ausnehmungen als Gewebeaufnahmeräume dienen können. Die Branchen sind komplett geschlossen, wenn die Versiegelungselektroden der beiden Branchen aufeinander liegen und die entsprechende Schließkraft von zum Beispiel 22N in Branchenmitte wirkt. Um elastische Verformungen der Branchen auszugleichen sind diese dabei so geformt, dass sich zuerst die Versiegelungselektrodenflächen am distalen Ende derselben berühren, bevor der vorhandene Dreieckspalt durch zunehmende Schließkraft und daraus resultierende elastische Verbiegung der Branchen allmählich geschlossen wird. Die Versiegelungselektroden der Branchen zueinander können so gestaltet sein, dass sie bei einem Abstand von zum Beispiel 0,1 mm einen Parallelspalt festlegen. Weil keine das weitere Schließen verhindernden Anschläge oder Abstandshalter vorgesehen sind, wird der Spalt beim weiteren Schließen am distalen Ende Null. Bei steigender Schließkraft verformen sich die Branchen dann zunehmend elastisch und der Spalt wird auch in einiger Distanz zum distalen Ende nahe Null.

In der Zeichnung sind Ausführungsbeispiele der Erfindung veranschaulicht. Es zeigen:
Fig. 1 ein Instrument zum Fassen, Koagulieren und Trennen von biologischem Gewebe angeschlossen an ein speisendes Gerät, in schematischer Darstellung,
Fig. 2 das Werkzeug des Instruments nach Figur 1, in vergrößerter perspektivischer Darstellung,
Fig. 3 das Werkzeug nach Figur 2 in gewendeter Position, in perspektivischer Darstellung vor dem Fixieren des Widerlagers,
Fig. 4 das Werkzeug nach Figur 3 mit fixiertem Widerlager,
Fig. 5 das Widerlager in perspektivischer Darstellung mit Blick auf seine Gewebeauflagefläche,
Fig. 6 das Widerlager nach Figur 5 mit Blick auf seine unterseitige Ausnehmung,
Fig. 7 eine schematische Skizze zur Veranschaulichung der elektrischen Versorgung des Instruments,
Fig. 8 die Kraft-Weg-Kennlinie (Federkennlinie) eines infinitesimal kleinen Segmentes des Widerlagers des Werkzeugs nach den Figuren 2 bis 4,
Figur 9A-9C das Werkzeug im Querschnitt, in verschiedenen Positionen beim Schließen, und
Fig. 10 eine abgewandelte Ausführungsform des Widerlagers in schematisierter perspektivischer Darstellung.

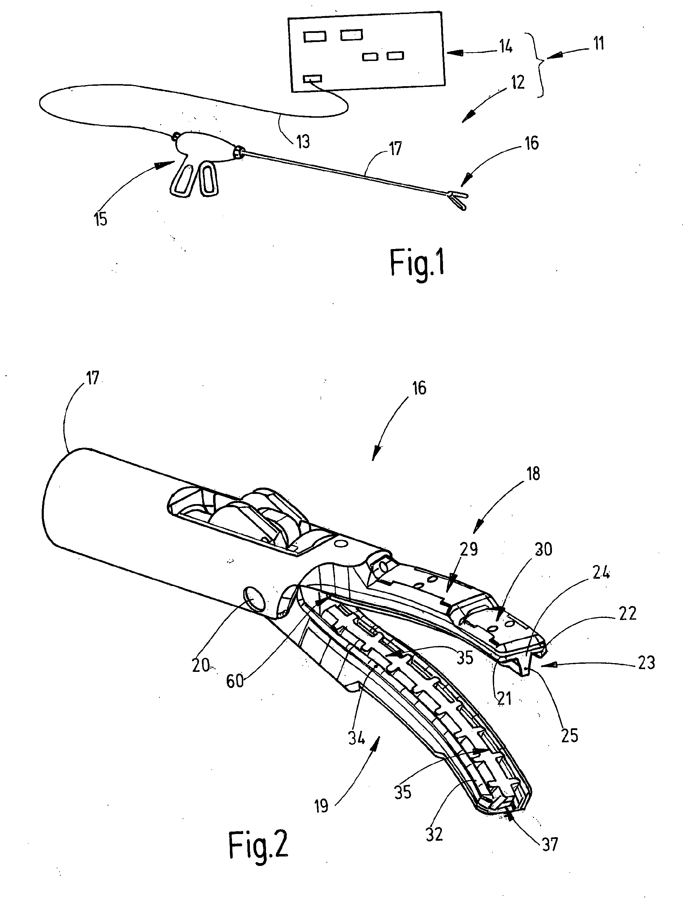

In Figur 1 ist eine Einrichtung 11 veranschaulicht, zu der ein Instrument 12 zum Fassen, Koagulieren und Trennen von biologischem Gewebe gehört, das über ein Kabel 13 mit einem speisenden Gerät 14 verbunden ist. Das Instrument 12 kann, wie dargestellt, ein Instrument zur laparoskopischen Chirurgie oder auch anderweitig ausgebildet sein. Zum Beispiel kann es als scherenartiges Instrument für die offenchirurgische Behandlung wie auch als Instrument zur endoskopischen Behandlung ausgebildet sein. Es kann außerdem einen Griff 15 zur manuellen Führung oder auch einen Anschluss zur maschinellen Führung (nicht dargestellt) aufweisen. Das Instrument 12 kann somit direkt von einem Anwender oder von einem Roboter geführt werden. Unabhängig davon weist es ein Werkzeug 16 auf, das in dem Ausführungsbeispiel nach Figur 1 an dem distalen Ende eines länglichen Schafts 17 des Instruments 15 angeordnet ist. Der Aufbau des Werkzeugs 16 ist nachfolgend erläutert:

Das gesondert in Figur 2 veranschaulichte Werkzeug 16 weist eine erste, in Figur 2 obere Branche 18 und eine zweite, in Figur 2 untere Branche 19 auf, die an einem Gelenk 20 miteinander verbunden sind, so dass zumindest eine der Branchen 18, 19 gegen die andere geschwenkt werden kann. Im Ausführungsbeispiel nach Figur 2 ist die untere Branche beweglich. Alternativ können beide Branchen 18, 19 oder auch lediglich die obere Branche 19 beweglich ausgebildet sein. Nachdem die räumliche Orientierung der Branchen 18, 19 von der Position des Instruments 15 abhängt, wird die obere Branche 18 nachfolgend als erste Branche und die untere Branche 19 nachfolgend als zweite Branche bezeichnet.

Die erste Branche 18 besteht vorzugsweise aus einem elektrisch leitenden Material, insbesondere Metall, und weist einen U-förmigen Querschnitt auf. Entsprechend ist zwischen zwei zueinander im Abstand verlaufenden Schenkeln 21, 22 ein nutartiger Zwischenraum ausgebildet, in dem ein Schneidelement 23 angeordnet ist. Dieses besteht aus einem elektrisch isolierenden Material, beispielsweise Kunststoff oder Keramik. Es weist einen in seiner Grundform etwa T-förmigen Querschnitt auf. Von einem unteren plattenartigen Fußabschnitt 24 ragt ein mittlerer Wandabschnitt 25 auf, der an seinem schmalen, der anderen Branche 19 zugewandten Seite eine Schneidelektrode 26 trägt. Diese kann durch ein Metallblech gebildet sein, das in den Wandabschnitt 25 eingefügt und dort gehalten ist. Die Schneidelektrode 26 liegt nur an der der anderen Branche 19 zugewandten Seite, ihrer Schneidefläche frei und überragt hier die schmale Stirnseite des Wandabschnitts 25 nicht oder lediglich um wenige Zehntel-Millimeter.

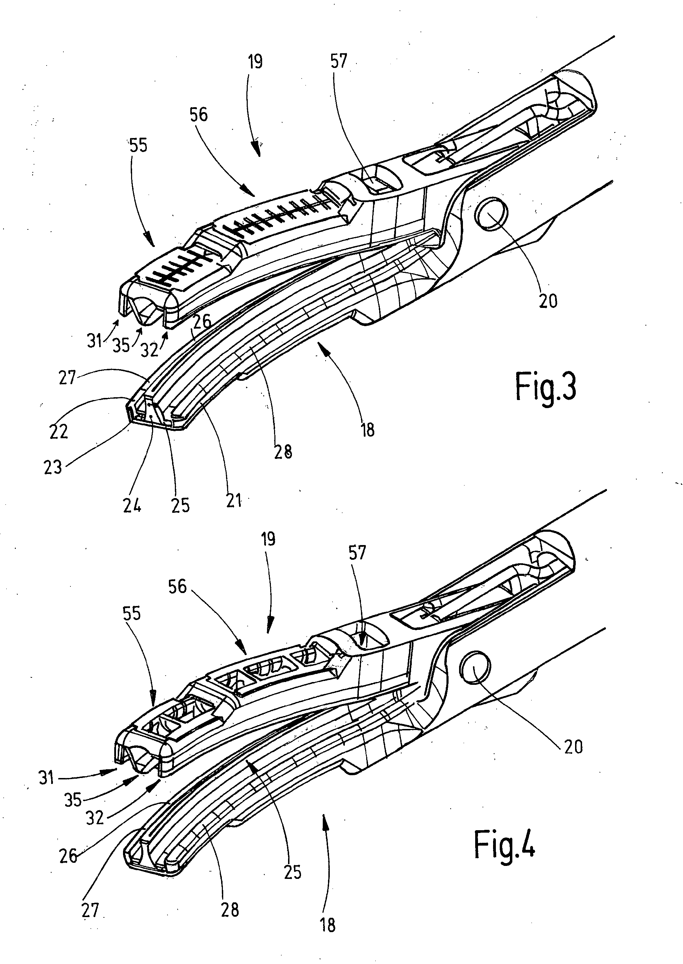

Die beiden Schenkel 21, 22 der Branche 19 werden durch jeweils zwei Seitenflächen, eine Schmalseite und eine quer zu dieser Schmalseite angeordnete Endfläche gebildet. Jeder Schenkel 21, 22 umfasst eine erste innere Seitenfläche und eine zweite äußere Seitenfläche. Die beiden ersten inneren Seitenflächen der Schenkel 21, 22 bilden zusammen mit einer Bodenfläche die Innenform des U-förmigen Querschnitts der Branche, also die Branchenaußenseite. Die beiden langen äußeren Seitenflächen bilden zusammen mit einer Rückenfläche die Außenform des U-förmigen Querschnitts der Branche.. Eine Ebene, die an beiden Schmalseiten der Schenkel 21, 22 aufliegt ist im Wesentlichen parallel zur Schneidefläche der Schneideelektrode 26 angeordnet. Die Versiegelungselektroden 27, 28 der Branche 19 sind im Wesentlichen an der Schmalseite in einem Winkel, vorzugsweise einem spitzen Winkel bezüglich der ersten Seitenfläche eines Schenkels 21, 22 angeordnet. Der Übergang einer Versiegelungselektrode 27, 28 in die erste und in die zweite Seitenfläche der Schenkel 21, 22 ist gerundet ausgebildet. Dabei kann sich der Radius der beiden Übergänge voneinander unterscheiden. Vorzugsweise ist der Radius des Übergangs einer Versiegelungselektrode 27, 28 zur ersten inneren Seitenfläche geringer, besonders vorzugsweise 4 x kleiner als der Radius des Übergangs einer Versiegelungselektrode zur zweiten äußeren Seitenfläche des Schenkels 21, 22. Die Versiegelungselektroden 27, 28, die an der Schmalseite der Schenkel angeordnet sind, reichen über die vorbeschriebenen Radien bis in die erste und die zweite Seitenfläche der Schenkel 21, 22. Das Werkzeug 16 weist mindestens eine Versiegelungselektrode 27, vorzugsweise jedoch zwei derartige erste Versiegelungselektroden auf, zwischen denen das Schneidelement 23 mit der Schneidelektrode 26 angeordnet ist. Zur Verbesserung der Übersichtlichkeit sind das Schneidelement 23 mit der Schneidelektrode 26 und die Versiegelungselektroden 27, 28 in den Figuren 3 und 4 veranschaulicht. Wie Figur 2 zeigt, kann das Schneidelement 23 in der Branche 18 formschlüssig verankert, und beispielsweise durch entsprechende Befestigungsbleche 29, 30 gegen das Austreten an der Branchenrückseite gesichert sein. Das Schneidelement 23 ist in diesem Fall an der ersten Branche 18 starr, d.h. unbeweglich gelagert. Eine bewegliche Lagerung ist jedoch bei jeder Ausführungsform als Abwandlung möglich. Auch das Schneideelement 23 selbst kann eine Bewegung ermöglichen oder nachgiebig oder elastisch ausgebildet sein.

Die zweite Branche 19 weist ähnlich wie die erste Branche und wie aus Figur 2 erkennbar einen U-förmigen Querschnitt mit Schenkeln 31, 32 auf, wobei an den Schmalseiten der beiden Schenkel 31, 32 des U-Querschnitts zweite Versiegelungselektroden 33, 34 angeordnet sind. Die Versiegelungselektroden 33, 34 der zweiten Branche 19 sind im geschlossenen Zustand des Instruments komplementär zu den Versiegelungselektroden 27, 28 der ersten Branche 18 angeordnet, was dazu führt, dass der spitze Winkel zwischen den Versiegelungselektroden 33, 34 und der zweiten Seitenfläche gebildet wird. Ansonsten gilt die Beschreibung der Versiegelungselektroden 27, 28 auch für die Versiegelungselektroden 33, 34 entsprechend. Die Versiegelungselektroden 33, 34 können, wie auch die ersten Versiegelungselektroden 27, 28, jeweils mit einer Reihe von isolierenden Feldern versehen sein, wobei die isolierenden Felder der ersten Versiegelungselektroden 27, 28 so gegen die isolierenden Felder der zweiten Versiegelungselektroden 33, 34 in Elektrodenlängsrichtung versetzt sind, dass ein elektrischer Kurzschluss bei Berührung der Branchen 18, 19 ausgeschlossen ist.

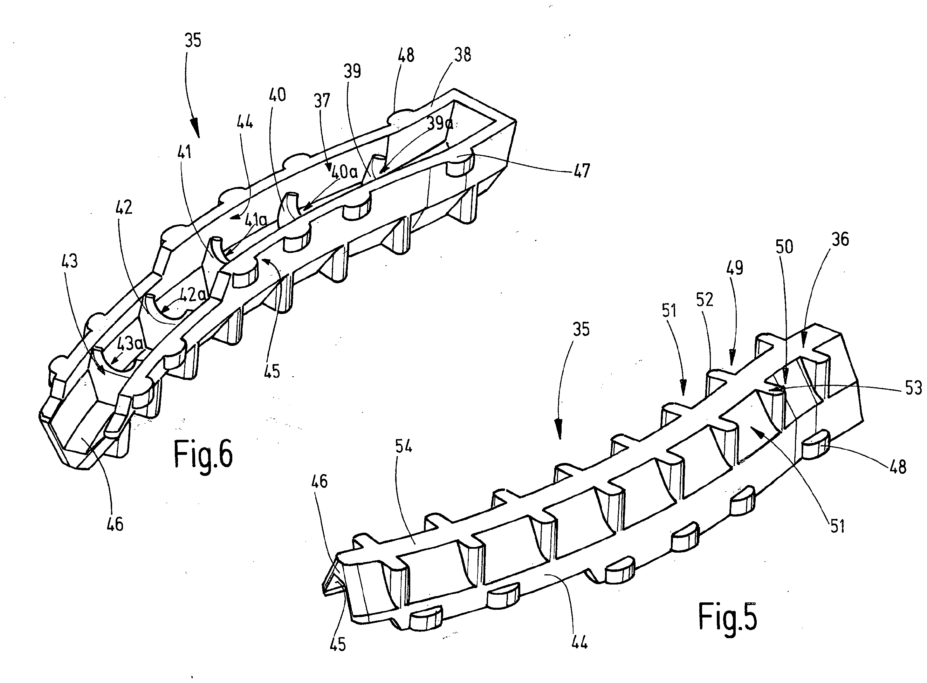

Zwischen den beiden Schenkeln 31, 32 der zweiten Branche 19 ist ein nutartiger Freiraum ausgebildet, in dem ein Widerlager 35 angeordnet ist, das vorzugsweise als gesondertes Element gefertigt ist. Das Widerlager 35 ist in den Figuren 5 und 6 gesondert und vergrößert veranschaulicht. Es ist vorzugsweise aus einem elastischen Werkstoff wie beispielsweise einem Elastomer, Silikon, Silikongummi, Gummi oder dergleichen, ausgebildet. An seiner der Schneidelektrode 26 zugewandten Seite weist das Widerlager 25 eine ebene oder leicht gewölbte Gewebeauflagefläche 36 auf. Im nicht komprimierten Zustand des Widerlagers ist die Gewebeauflagefläche 36 im Wesentlichen parallel zu einer auf den Schmalseiten der Schenkel 31, 32 liegenden Ebene angeordnet. Die Gewebeauflagefläche 36 weist mittig einen streifenförmigen Schneidelektroden-Auftreffbereich 54 auf, der der Schneidelektrode 26 unmittelbar gegenüberliegt und an dem die Schneidelektrode 26 beim gelenkförmigen Schließen des Werkzeugs 16 zur Berührung kommt.

Das Widerlager 35 ist vorzugsweise einstückig ausgebildet, beispielsweise als Spritzguss-, Spritzpress, Press- oder Sinterteil, d.h. es besteht nahtlos aus einem einheitlichen Material. Vorzugsweise weist es auf seiner der Gewebeauflagefläche 36 abgewandten Seite mindestens eine Ausnehmung 37 auf (Figur 6), die eine Grundfläche 38 des Widerlagers 35 durchsetzt und somit nach außen offen ist. Die Ausnehmung 37 kann wie aus Figur 6 ersichtlich als eine längliche durch Querwände 39, 40, 41, 42, 43 in Kammern unterteilte Nut oder dergleichen, ausgebildet sein. Die Querwände 39, 40, 41, 42, 43 können in gleichen oder unterschiedlichen Abständen zueinander angeordnet sein. Sie können zu der Hauptgrundfläche 38 rechtwinklig oder auch geneigt angeordnet sein.

Die Querwände 39, 40, 41, 42, 43 können einen geschwächten, d.h. mit geringerer Wandstärke versehenen Abschnitt aufweisen. Auch ist es möglich, die Querwände 39 bis 43, wie dargestellt, bodenseitig jeweils mit einer vorzugsweise gerundeten, maulartigen Ausnehmung 39a, 40a, 41a, 42a, 43a zu versehen. Über Größe und Form der Ausnehmung 39a, 40a, 41a, 42a, 43a lässt sich die Steifigkeit jeder Querwand 39 bis 43 gezielt festlegen und somit die Nachgiebigkeit des Widerlagers 35 insgesamt beeinflussen. Auch ist es möglich, die Querwände 39 bis 43 rechtwinklig zu der Grundfläche 38 oder auch in einer Neigung zu dieser anzuordnen. Damit, und mit der Größe und Form der Ausnehmung 39a, 40a, 41a, 42a, 43a, lässt sich nicht nur die Nachgiebigkeit insgesamt, sondern auch die Form der erzielbaren Federkennlinie und die Verformung steuern.

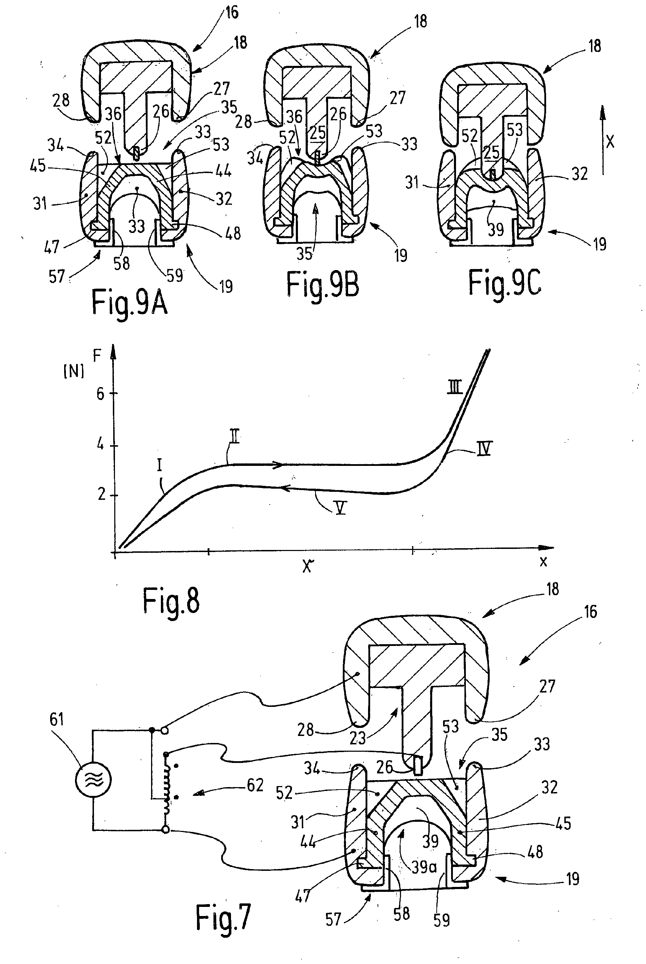

Die Ausnehmung 37 ist in Längsrichtung seitlich von Wänden 44, 45 begrenzt, die zusammen mit einem die Gewebeauflagefläche 36 tragenden Dachabschnitt 46 einen trapezförmigen Abschnitt der Nut bzw. Ausnehmung 37 festlegen. Der Dachabschnitt 46 bildet zugleich den Boden der nutartigen Ausnehmung 37. Die den trapezförmigen Teil der Ausnehmung 37 begrenzenden Teile der Seitenwände 44, 45 sind vorzugsweise aufeinander zu geneigt, und im Querschnitt gerade ausgebildet wie Figur 7 zeigt. Im Ausführungsbeispiel gemäß Figur 7 weisen die Seitenwände einen konstanten Querschnitt auf. Die Querschnittsform hat einen Einfluss auf die Federkennlinie des Widerlagers 35 und kann entsprechend der Anforderungen andere Formen einnehmen.

Weiter können die Seitenwände 44, 45 zueinander parallel verlaufende Abschnitte aufweisen, die Füße 47, 48 zur Befestigung des Widerlagers 35 in der Branche 19 tragen. Den Füßen 47, 48 sind in der Branche 19 vorgesehene komplementäre Ausnehmungen zugeordnet, in die die Füße 47, 48 fassen, um darin gesichert zu sein.

Das Widerlager 35 kann auch alternativ als Silikon/Metall Verbundteil ausgebildet sein. Dabei können andere Befestigungsmittel als die oben beschriebenen Füße 47, 48 vorgesehen sein. Diese Befestigungsmittel können aus einem anderen Werkstoff als dem elastischen Werkstoff, der die Federkennlinie des Widerlagers prägt, gebildet sein. Trotzdem kann das Widerlager als einstückiges Bauteil betrachtet werden. Weist das Widerlager 35 Befestigungsmittel anderer Art auf, können die oben beschriebenen Ausnehmungen in der Branche 19 anders gestaltet sein, oder komplett fehlen.

Das Widerlager 35 kann außer der Ausnehmung 37 weitere Ausnehmungen aufweisen. Zum Beispiel können solche Ausnehmungen 49, 50, 51 usw. an der der Grundfläche 38 abgewandten Seite des Widerlagers 35 ausgebildet sein. Solche Ausnehmungen 49 bis 51 können die Gewebeauflagefläche 36 durchsetzend ausgebildet sein und als sich zu beiden Seiten des Widerlagers 35 über dessen gesamte Länge als Reihe erstreckend angeordnet sein. Zwischen jeweils zwei benachbarten Ausnehmungen 49, 51 bzw. 50, 52, können Zentrierstege 52, 53 ausgebildet sein, die vorzugsweise so dimensioniert sind, dass sie im Ausgangszustand, im nicht verformten Zustand, an der Innenflanke des jeweiligen Schenkels 31, 32 der Branche 19 anliegen. An der Oberseite schließen die Zentrierstege 52, 53 sowie die zwischen den weiteren Ausnehmungen vorgesehenen Zentrierstege vorzugsweise eben mit der Gewebeauflagefläche 36 ab. Die Zentrierstege 52, 53 haben eine doppelte Zentrierfunktion: im unbelasteten Zustand zentrieren sie das Widerlager 35 zwischen den Versiegelungselektroden 33, 34 (Figur 7 und 9A) und im geschlossenen Zustand des Werkzeugs 16 zentrieren sie das Widerlager 35 in Bezug auf das Schneidelement 23 (Figur 9C). Es ist auch möglich, dass die Zentrierstege 52, 53 in Richtung der Schenkel 31, 32 abgerundet oder schräg ausgebildet sind um dadurch mehr Raum für die Aufnahme von Gewebe innerhalb der geschlossenen Branchen 18, 19 zu bilden ohne die oben beschriebene Zentrierfunktionen maßgeblich zu verändern.

Die Gewebeauflagefläche 36 besteht somit aus dem schmalen streifenförmigen Schneidelektroden-Auftreffbereich 54, der sich entlang der Länge des Widerlagers 35 erstreckt, und aus den Oberseiten der sich zu beiden Seiten befindlichen Zentrierstege 52, 53. Zwischen den Zentrierstegen 52, 53 u.a. sind jeweils paarweise Ausnehmungen angeordnet, die den Ausnehmungen 49 bis 51 entsprechen und im Querschnitt dreieckförmig sein können.

Das insoweit beschriebene Widerlager 35 sitzt formschlüssig in der Branche 19 wie es Figur 7 nahelegt. Zur Befestigung darin, können aus Figur 3 und 4 ersichtliche Halteelemente 55, 56, 57 dienen, die an der Rückseite der Branche 19 anliegen und mit entsprechenden Laschen 58, 59 durch in der Branche 19 vorgesehene Ausnehmungen greifen, um das Widerlager 35 zu fixieren. Figur 3 veranschaulicht die Halteelemente 55, 56, 57 vor dem Fixieren und Figur 4 sowie 7 nach dem Fixieren. In Figur 7 sind zwei Laschen 58, 59 des Halteelements 57 beispielhaft in fixiertem Zustand veranschaulicht.

Das Widerlager 35 und das Schneidelement 23 bilden gemäß der Darstellung in Figur 2 außerdem einen axialen Gewebeanschlag. Dazu weist das Schneidelement 23 vorzugsweise eine Gewebeanschlagnase mit einer Gewebeanlagefläche 60 auf, die vorzugsweise einem zur Gelenkachse konzentrischen Kreisbogen folgt. Die entsprechende an dem proximalen Ende ausgebildete Fläche des Widerlagers 35 weist eine komplementär passende Gegenfläche auf, die vorzugsweise spaltfrei an der Gewebeanlagefläche 60 anliegt.

Die elektrische Beschaltung des Werkzeugs 16 zeigt Figur 7. Eine geeignete elektrische Quelle, beispielsweise in Gestalt eines HF-Generators 61, stellt elektrische Leistung, zum Beispiel in Form einer Wechselspannung mit mehreren Hundert kHz bereit. Diese Spannung kann vorzugsweise direkt an die Branchen 18, 19 angelegt und somit zwischen den ersten Versiegelungselektroden 27, 28 und den zweiten Versiegelungselektroden 33, 34 wirksam werden. Ein von dem HF-Generator 61 gespeister Transformator 62 kann dazu genutzt werden, die HF-Spannung hoch zu transformieren und die Schneidelektrode 26 zu speisen.

Das Instrument 12 und insbesondere das Werkzeug 16 arbeiten wie folgt:

Im Folgenden wird ein beispielhafter Querschnitt des sich gelenkig schließenden Maulteils betrachtet. Im offenen oder ohne Kraft geschlossenen Zustand des betrachteten Querschnitts hat das Werkzeug 16 den Zustand nach Figur 7 und 9A. Die Schneidelektrode 26 liegt ohne Kraft an der Gewebeauflagefläche 36 an oder hält einen Abstand zu dieser ein. Zwischen den Branchen 18, 19 gefasstes Gewebe wäre in diesem Querschnitt wenig oder nicht deformiert.

Werden die Branchen 18, 19 im betrachteten Querschnitt nun weiter aufeinander zu bewegt, wie es in Figur 9B ohne gefasstes Gewebe veranschaulicht ist, nähern sich die ersten Versiegelungselektroden 27, 28 den zweiten Versiegelungselektroden 33, 34 an. Dazwischen gefasstes Gewebe würde geklemmt und deformiert. Außerdem wird das Gewebe bestromt, und dadurch geschnitten und koaguliert. Dabei stellt das Widerlager 35 durch seine Elastizität sicher, dass trotz Gewebeschrumpfung stets Gewebe an der Scheideelektrode anliegt bis dieses Gewebe vollständig geschnitten ist.

Befindet sich kein Gewebe zwischen den Branchen drückt die Schneidelektrode 26 auf die Gewebeauflagefläche 36 und verformt das Widerlager 35. Währenddessen steigt die von dem Widerlager 35 der Schneidelektrode 26 entgegen gerichtete Kraft, wie es in Figur 8 in einem ersten Abschnitt I der dargestellten Kurve veranschaulicht ist allmählich an, um dann mit dem Abschnitt II ein Plateau zu erreichen. Diese Darstellung des Diagramms gemäß Figur 8 bezieht sich auf eine infinitesimal kleines Querschnittsegment der Branchen des Instruments. Die im Diagramm angegebenen Kräfte beziehen sich auf über die gesamte Länge des Widerlagerelements über, alle infenitesimalen Elemente hochgerechnete (z.B. aufsummierten) Werte. Alternativ kann diese Kennlinie auch ermittelt werden, indem die erste Branche und die zweite Branche in einer parallelen Ausrichtung zueinander aufeinander zu bewegt (geschlossen) werden.

Beim weiteren Zusammenführen der Branchen 18, 19 im betrachteten Querschnitt verformt sich das Widerlager 35, wie in Figur 9C dargestellt. Die Stege 52, 53 sind von den Innenseiten der Schenkel 31, 32 abgehoben und legen sich an die Seitenflächen des Wandabschnitts 25 an. Die Seitenwände 44, 45 knicken dabei, wie ein Vergleich der Figuren 9A bis 9C zeigt, zunehmend ein und die Querwand 39 weicht entsprechend nach unten aus. Schließlich fahren die Branchen aufeinander auf bzw. kommt es zum Schluss der Versiegelungselektroden 27, 28 und 33, 34 und das Maulteil ist geschlossen. Ab hier findet keine weitere Komprimierung des Widerlagers statt, außer es befindet sich noch Gewebe im Maulteil. In diesem Falle kann ein Teil des Widerlagers 35, z.B. die Querwand 39, in Kontakt mit wenigstens einem der Haltebleche 55 bis 57 kommen. In der Folge darauf ist ein Kraftanstieg III nach Durchlaufen des Plateaus II in Figur 8 zu beobachten.

Während des Schneidens des Gewebes bzw. wenn das geschlossene Maulteil wieder geöffnet wird, richtet sich das deformierte Widerlager 35 wieder auf. Die Rückstellkraft des Widerlagers 35 im betrachteten Querschnitt nimmt wieder ab, siehe Kurvenverlauf IV. Sobald alle Teile des Widerlagers 35, die auf den Halteblechen 55 bis 57 zur Auflage kamen, von diesen wieder abgehoben sind, findet eine Verformung bei relativ konstanter Kraft statt, was dem Kurvenzug V entspricht. Die von dem Widerlager 35 gegen die Schneidelektrode 26 aufgebrachte Kraft auf diesem rücklaufenden Plateaubereich V läge bei Betrachtung eines idealisierten parallelen Schlusses im Bereich von 2N bis 4Nin Branchenmitte. Es kann eine Hysterese aufgrund des Energieverlustes im elastischen Material im Kraft-Weg-Verlauf auftreten.

Ist beim Maulteilschließen zwischen der Schneidelektrode 26 und der Gewebeauflagefläche 36 zu schneidendes Gewebe vorhanden, verformt sich das Widerlager 35 bereits bevor die Schneidelektrode 26 mit der Gewebeauflagefläche 36 in Kontakt kommt. Wird die Aktivierung gestartet, finden der Schneide- und der Versiegelungsprozess gleichzeitig statt. Während des Schneide- und des Versiegelungsprozesses treten an unterschiedlichen Stellen unterschiedliche Gewebebewegungen auf. Wenn das zu schneidende Gewebe durch den Schneidevorgang allmählich nachgibt, nähert sich die Gewebeauflagefläche 36 mehr und mehr der Schneidelektrode 26 an. An dieser Stelle gibt die Rückstellkraft des Widerlagers 35 die Schneidekraft vor und sorgt im Wesentlichen für die Schneidebewegung. Bei geschlossenen Branchen 18, 19, mit oder ohne Gewebe, befindet sich das Widerlager 35 vollständig innerhalb der Außenform des Maulteils welches durch die Branchen 18, 19 gebildet ist. Das Widerlager überragt bei geschlossenem Maulteil nirgends die Branchenaußenseiten. Das Widerlager reicht bei geschlossenen Branchen 18, 19 weder im entspannten noch komprimierten Zustand über die Rückenfläche der Branche 19, in der es gehalten ist, hinaus.

Die insgesamt zwischen den Branchen 18, 19 anliegende Schließkraft ist hingegen bedeutend höher als die Kraft zwischen Widerlager 35 und Schneidelektrode 26 und kann zum Beispiel 10N bis 30N, beispielsweise 22N in Branchenmitte betragen. Unabhängig davon stellt das Widerlager 35 an der Schneidelektrode 26 lediglich eine Kraft von 2N bis 8N ein, so dass die restliche Kraft zwischen der Versiegelungselektroden 27, 33 bzw. 28, 34 wirksam wird. Auf diese Weise lässt sich beides erreichen: Eine sichere Versiegelung bzw. Koagulation aufgrund hoher Kräfte und ein sauberer im Wesentlichen rein elektrisch durchgeführter Schnitt. Die oben angegebenen Werte der Schließkraft sind abhängig von der Auslegung, insbesondere der Größe der Branchen des chirurgischen Instruments. Veränderte geometrische Bedingungen beispielsweise für eine offenchirurgische oder maschinengesteuerte Anwendung können andere Schließkraftwerte ergeben.

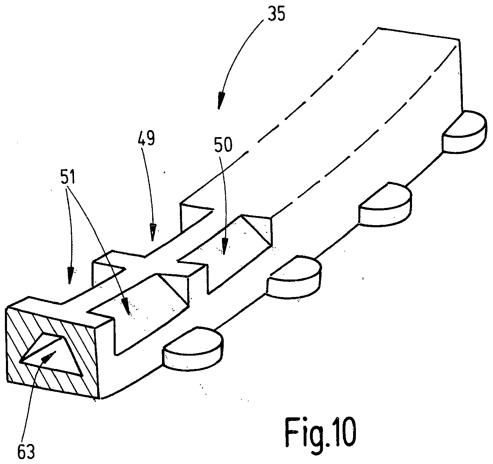

Das Widerlager 35 kann anstelle der offenen Ausnehmung 37 auch eine Hohlkammer 63 aufweisen, wie es Figur 10 veranschaulicht. Zusätzlich können Ausnehmungen 49 bis 51 vorgesehen sein. Solche Ausnehmungen können jedoch auch entfallen. Die Hohlkammer 63 kann rund, trapezförmig, rechteckig, sternförmig oder in ähnlichen Formen ausgebildet sein.

Anstelle einzelner großer Kammern ist es auch möglich, eine Vielzahl kleiner Kammern vorzusehen, beispielsweise indem der Körper des Widerlagers 35 generell oder zonenweise als offenzelliger oder geschlossenzelliger Schaum ausgebildet ist.

Bei einem erfindungsgemäßen Werkzeug 16 zur Koagulation und Dissektion von biologischem Gewebe ist ein Widerlager 35 vorgesehen, das mindestens eine nach außen offene Ausnehmung 37 und/oder eine oder mehrere Hohlkammern 63 aufweist. Die Ausnehmung 37 oder die Hohlkammer 63 sind so gestaltet, dass das Widerlager 35, bei Betrachtung eines infinitesimal kleinen Querschnittsegments, beim Zusammendrücken und Reexpandieren eine Kraft-Weg-Federkennlinie mit einem plateauartigen Bereich II, V ausbildet. Auf diesem Plateau II, V erbringt das Widerlager 35, bei Betrachtung eines idealisiert parallelen Schlusses von Widerlager und Schneidelektrode, vorzugsweise eine Kraft zwischen 2N und 4N. Der Wegbereich x, in dem die Federkennlinie das Plateau II, IV zeigt, ist vorzugsweise so groß wie die Dicke des zu durchtrennenden Gewebes, und liegt beispielsweise im Bereich von 0,2 bis 1,5 mm vorzugsweise ist die Dicke 1 mm, besonders vorzugsweise 0,5 mm. Damit wird erreicht, dass die auf das Gewebe ausgeübte Kraft während des Schneidvorgangs im wirksamen Arbeitsbereich im Wesentlichen konstant bleibt.

**Bezugszeichen:**

| | |
|---|---|
| 11 | Einrichtung |
| 12 | Instrument |
| 13 | Kabel |
| 14 | Gerät zu Speisung des Instruments |
| 15 | Griff |
| 16 | Werkzeug |
| 17 | Schaft |
| 18 | erste (obere) Branche |
| 19 | zweite (untere) Branche |
| 20 | Gelenk |
| 21, 22 | Schenkel des U-Querschnitts der ersten Branche 18 |
| 23 | Schneidelement |
| 24 | Fußabschnitt |
| 25 | Wandabschnitt |
| 26 | Schneidelektrode |
| 27, 28 | erste Koagulations-, Versiegelungselektrode |
| 29, 30 | Befestigungsbleche |
| 31, 32 | Schenkel des U-Querschnitts der zweite Branche 19 |
| 33, 34 | zweite Koagulations-, Versiegelungselektrode |
| 35 | Widerlager |
| 36 | Gewebeauflagefläche |
| 37 | Ausnehmung |
| 38 | Grundfläche |
| 39-43 | Querwände |
| 39a-43a | Ausnehmungen der Querwände 39, 40, 41, 42, 43 |
| 44, 45 | Seitenwände/ Boden der Ausnehmung 37 |
| 46 | Dachabschnitt, Boden |
| 47, 48 | Füße |
| 49-51 | Ausnehmungen |
| 52, 53 | Zentrierstege |
| 54 | Schneidelektroden-Auftreffbereich |
| 55-57 | Halteelement |
| 58, 59 | Laschen |
| 60 | Gewebeanlagefläche |
| 61 | HF-Generator |
| 62 | Transformator |
| 63 | Hohlkammer |

## Patentansprüche

1. Instrument (12) zum Fassen, Koagulieren und Trennen von biologischem Gewebe,
mit einer ersten Branche (18), an der mindestens eine erste Versiegelungselektrode (27, 28) vorgesehen ist,
mit einer zweiten Branche (19), an der mindestens eine zweite Versiegelungselektrode (33, 34) vorgesehen ist,
wobei wenigstens eine der Branchen (18, 19) beweglich angeordnet ist, um die Branchen (18, 19) aufeinander zu und voneinander weg zu bewegen,
mit einer Schneidelektrode (26), die an einer der Branchen (18, 19) angeordnet ist,
mit einem Widerlager (35), das an einer der Branchen (18, 19) angeordnet und aus einem elastischen Material ausgebildet ist und das eine der Schneidelektrode (26) zugewandte Gewebeauflagefläche (36) aufweist,
**dadurch gekennzeichnet,**
**dass** das Widerlager (35) mindestens eine nach außen offene, die Gewebeauflagefläche (36) durchsetzend angeordnete Ausnehmung (49 - 51), die nicht allseits von dem Widerlager (35) umschlossen ist, sondern als Öffnung an dessen Oberfläche zutage tritt, aufweist und dass die wenigstens eine Ausnehmung (49 - 51) außerhalb eines Schneidelektroden-Auftreffbereichs (54) der Gewebeauflagefläche (36) angeordnet ist, auf dem die Schneidelektrode bei geschlossenen Branchen (18, 19) aufliegt.

2. Instrument nach Anspruch 1, **dadurch gekennzeichnet, dass** die Ausnehmung (37) als eine der Gewebeauflagefläche (36) abgewandte Nut ausgebildet ist.

3. Instrument nach einem der vorstehenden Ansprüche, **dadurch gekennzeichnet, dass** die Ausnehmung (37) den Querschnitt des Widerlagers ganz oder teilweise durchdringend angeordnet ist.

4. Instrument nach Anspruch 2, **dadurch gekennzeichnet, dass** die Nut einen sich zu ihrem Boden (46) hin verjüngenden Querschnitt aufweist.

5. Instrument nach einem der vorstehenden Ansprüche, jedoch zumindest Anspruch 2, **dadurch gekennzeichnet, dass** in der Nut mindestens eine Querwand (39 - 43) angeordnet ist.

6. Instrument nach einem der vorstehenden Ansprüche, **dadurch gekennzeichnet, dass** das Widerlager (35) seitlich vorstehende Füße (47, 48) aufweist, denen komplementäre Ausnehmungen in der Branche (18, 19) zugeordnet sind.

7. Instrument nach einem der vorstehenden Ansprüche, **dadurch gekennzeichnet, dass** die das Widerlager (35) tragende Branche (19) eine dem Widerlager (35) abgewandte Ausnehmung aufweist, an der mindestens ein Halteelement (55 - 57) zur Fixierung des Widerlagers (35) angeordnet ist.

8. Instrument nach einem der vorstehenden Ansprüche, **dadurch gekennzeichnet, dass** das Widerlager (35) im entspannten oder komprimierten Zustand, bei geschlossenen Branchen (18, 19) nicht über die dem Widerlager (35) abgewandte Ausnehmung der Branche (19) hinaus reicht.

9. Instrument nach Anspruch 7, oder nach Ansprüchen 7 und 8, **dadurch gekennzeichnet, dass** das Halteelement (55 - 57) Laschen (58, 59) aufweist, die sich in die wenigstens eine Ausnehmung (37) des Widerlagers (35) erstreckend angeordnet sind, um die seitlichen Füße (47, 48) in den komplementären Ausnehmungen zu halten.

10. Instrument nach Anspruch 1, **dadurch gekennzeichnet, dass** die Ausnehmungen (49 - 51) vorzugsweise zu beiden Seiten des Schneidelektroden-Auftreffbereichs (54) angeordnet sind.

11. Instrument nach Anspruch 1, **dadurch gekennzeichnet, dass** mehrere Ausnehmungen (49 - 51) entlang einer Seite des Schneidelektroden-Auftreffbereichs (54) angeordnet und voneinander durch Zentrierstege (52, 53) getrennt sind.

12. Instrument nach Anspruch 11, **dadurch gekennzeichnet, dass** die Zentrierstege (52, 53) an Seitenflächen einer in der Branche (19) ausgebildeten Nut anliegend ausgebildet sind.

13. Instrument nach einem der vorstehenden Ansprüche, **dadurch gekennzeichnet, dass** das Widerlager (35) bei Betrachtung eines nur sehr kurzen, infinitesimal kleinen Querschnittsegmentes eine nichtlineare Federkennlinie mit einem Plateau aufweist.

14. Instrument nach einem der vorstehenden Ansprüche, **dadurch gekennzeichnet, dass** das elastische Material ein Material aus der Gruppe der Silikone ist.

15. Instrument nach Anspruch 1, **dadurch gekennzeichnet, dass** das Widerlager eine Hohlkammer (63) aufweist.

## Claims

1. Instrument (12) for holding, coagulating and separating biological tissue,
with a first branch (18) on which at least one first sealing electrode (27, 28) is provided,
with a second branch (19) on which at least one second sealing electrode (33, 34) is provided,
wherein at least one of the branches (18, 19) is arranged movably so that the branches (18, 19) can be moved towards each other and away from each other,
with a cutting electrode (26) which is arranged on one of the branches (18, 19),
with an abutment (35) which is arranged on one of the branches (18, 19) and made from an elastic material, and which has a tissue support face (26) facing the cutting electrode (26),
**characterised in that**
the abutment (35) has at least one recess (49 - 51) which is open towards the outside and extends through the tissue support face (36), and which is not surrounded by the abutment (35) on all sides but opens onto its surface as an orifice, and that the at least one recess (49 - 51) is arranged outside a cutting electrode impact region (54) of the tissue support face (36) on which the cutting electrode lies when the branches (18, 19) are closed.

2. Instrument according to claim 1, **characterised in that** the recess (37) is formed as a groove facing away from the tissue support face (36).

3. Instrument according to any of the preceding claims, **characterised in that** the recess (37) is arranged so as to extend fully or partially through the cross-section of the abutment.

4. Instrument according to claim 2, **characterised in that** the groove has a cross-section tapering towards its base (46).

5. Instrument according to any of the preceding claims but at least claim 2, **characterised in that** at least one transverse wall (39 - 43) is arranged in the groove.

6. Instrument according to any of the preceding claims, **characterised in that** the abutment (35) has laterally protruding feet (47, 48) which are assigned to complementary recesses in the branch (18, 19).

7. Instrument according to any of the preceding claims, **characterised in that** the branch (19) carrying the abutment (35) has a recess which faces away from the abutment (35) and on which at least one holding element (55 - 57) is arranged for fixing the abutment (35).

8. Instrument according to any of the preceding claims, **characterised in that** in relaxed or compressed state, when the branches (18, 19) are closed, the abutment (35) does not extend beyond the recess of the branch (19) facing away from the abutment (35).

9. Instrument according to claim 7 or claims 7 and 8, **characterised in that** the holding element (55 - 57) has tabs (58, 59) which are arranged extending into the at least one recess (37) of the abutment (35) in order to hold the lateral feet (47, 48) in the complementary recesses.

10. Instrument according to claim 1, **characterised in that** the recesses (49 - 51) are preferably arranged on both sides of the cutting electrode impact region (54).

11. Instrument according to claim 1, **characterised in that** several recesses (49 - 51) are arranged along one side of the cutting electrode impact region (54) and are separated from each other by centring webs (52, 53).

12. Instrument according to claim 11, **characterised in that** the centring webs (52, 53) are formed so as to lie against side faces of a groove formed in the branch (19).

13. Instrument according to any of the preceding claims, **characterised in that** when only a very short and infinitesimally small cross-sectional segment is considered, the abutment (35) has a non-linear spring curve with a plateau.

14. Instrument according to any of the preceding claims, **characterised in that** the elastic material is material from the group of silicones.

15. Instrument according to claim 1, **characterised in that** the abutment comprises a hollow chamber (63).

## Revendications

1. Instrument (12) destiné à la préhension, la coagulation et la séparation de tissus biologiques,
comprenant un premier mors (18) sur lequel il est prévu au moins une première électrode de scellement (27, 28),
comprenant un deuxième mors (19) sur lequel il est prévu au moins une deuxième électrode de scellement (33,34),
au moins un des mors (18, 19) étant disposé de façon mobile pour déplacer les mors (18, 19) l'un vers l'autre et les éloigner l'un de l'autre,
comprenant une électrode de coupe (26) qui est disposée sur l'un des mors (18, 19),
comprenant un élément de butée (35) qui est disposé sur l'un des mors (18, 19) et est réalisé à partir d'un matériau élastique et qui présente une surface d'appui de tissu (36) tournée vers l'électrode de coupe (26),
**caractérisé en ce que**
l'élément de butée (35) comporte au moins un évidement (49 à 51) ouvert vers l'extérieur qui est disposé de manière à traverser la surface d'appui de tissu (36) et qui n'est pas entouré sur tous les côtés par l'élément de butée (35) mais se présente sous forme d'ouverture à la surface de celui-ci, et **en ce que** l'évidement (49 à 51), au nombre d'au moins un, est disposé à l'extérieur d'une zone de contact de l'électrode de coupe (54) de la surface d'appui de tissu (36), sur laquelle l'électrode de coupe est en appui lorsque les mors (18, 19) sont fermés.

2. Instrument selon la revendication 1, **caractérisé en ce que** l'évidement (37) est réalisé sous la forme d'une rainure orientée dans le sens opposé à la surface d'appui de tissu (36).

3. Instrument selon l'une des revendications précédentes, **caractérisé en ce que** l'évidement (37) est disposé de manière à traverser totalement ou en partie la section transversale de l'élément de butée.

4. Instrument selon la revendication 2, **caractérisé en ce que** la rainure présente une section transversale qui diminue en direction de son fond (46).

5. Instrument selon l'une des revendications précédentes mais au moins selon la revendication 2, **caractérisé en ce qu'**au moins une paroi transversale (39 à 43) est disposée dans la rainure.

6. Instrument selon l'une des revendications précédentes, **caractérisé en ce que** l'élément de butée (35) présente des pattes (47, 48) faisant saillie latéralement, auxquelles sont associés des évidements complémentaires dans le mors (18, 19).

7. Instrument selon l'une des revendications précédentes, **caractérisé en ce que** le mors (19) portant l'élément de butée (35) présente un évidement qui est orienté dans le sens opposé à l'élément de butée (35) et sur lequel est disposé au moins un élément de maintien (55 à 57) pour la fixation de l'élément de butée (35).

8. Instrument selon l'une des revendications précédentes, **caractérisé en ce que**, à l'état relâché ou comprimé, lorsque les mors (18, 19) sont fermés, l'élément de butée (35) ne dépasse pas par rapport à l'évidement du mors (19) qui est détourné de l'élément de butée (35).

9. Instrument selon la revendication 7 ou selon les revendications 7 et 8, **caractérisé en ce que** l'élément de maintien (55 à 57) présente des ailes (58, 59) qui sont disposées de manière à s'étendre dans l'évidement (37), au nombre d'au moins un, de l'élément de butée (35), aux fins de maintenir les pattes (47, 48) latérales dans les évidements complémentaires.

10. Instrument selon la revendication 1, **caractérisé en ce que** les évidements (49 à 51) sont placés de préférence de part et d'autre de la zone de contact de l'électrode de coupe (54).

11. Instrument selon la revendication 1, **caractérisé en ce que** plusieurs évidements (49 à 51) sont disposés le long d'un côté de la zone de contact de l'électrode de coupe (54) et sont séparés les uns des autres par des parois de centrage (52, 53).

12. Instrument selon la revendication 11, **caractérisé en ce que** les parois de centrage (52, 53) sont réalisées de manière à être appliquées contre des surfaces latérales d'une rainure réalisée dans le mors (19).

13. Instrument selon l'une des revendications précédentes, **caractérisé en ce que**, en observant un segment de section transversale de dimensions infinitésimales et de très faible longueur, l'élément de butée (35) présente une courbe caractéristique de ressort non linéaire comportant un plateau.

14. Instrument selon l'une des revendications précédentes, **caractérisé en ce que** le matériau élastique est un matériau du groupe des silicones.

15. Instrument selon la revendication 1, **caractérisé en ce que** l'élément de butée présente une cavité (63).
